# EUROPEAN PATENT SPECIFICATION

(11) **EP 0 714 885 B1**
(45) Date of publication and mention of the grant of the patent: **01.09.1999**
(21) Application number: 95118060.3
(22) Date of filing: 16.11.1995
(51) Int. Cl.: C07C 237/04, C07C 231/12

(54) **Preparation process of aminoacetamide derivative**
Verfahren zur Herstellung von Aminoacetamid Derivaten
Procédé pour la préparation d'un dérivé aminoacétamide

(30) Priority: 29.11.1994 JP 31776794
(43) Date of publication of application: 05.06.1996
(73) Proprietor: Eisai Co., Ltd., Tokyo (JP)
(72) Inventor: Seki, Chiaki, Komaki-shi, Aichi Pref. (JP); Ishizawa, Shozo, Kashima-gun, Ibaraki Pref. (JP); Koiwa, Atsushi, Kashima-gun, Ibaraki Pref. (JP); Ogura, Toshimasa, Iwata-gun, Shizuoka Pref. (JP); Takaha, Motoshige, Ogasa-gun, Shizuoka Pref. (JP); Uchiyama, Manabu, Ogasa-gun, Shizuoka Pref. (JP)
(74) Representative: Hansen, Bernd, Dr. Dipl.-Chem.

(56) References cited:
- BE-A- 885 303
- FR-M- 5 423
- US-A- 5 128 465
- CHIMIE TH RAPEUTIQUE, vol. 1, no. 2, 1966, pages 100-102, XP002003865 R. RIPS ET AL. : "Structure chimique et activité pharmaceutique de dérivés de la glycine: Glycinamides"
- "Methoden der Organischen Chemie (Houben -Weyl) Band XI/1" 1957 , GEORG THIEME VERLAG , STUTTGART XP002003878 * page 956 - page 957 *
- Liebigs Ann. Chem. 721,19-24(1969)

## Description

### BACKGROUND OF THE INVENTION

### Field of the Invention:

The present invention relates to novel preparation processes of 2-aminoacetamide derivatives useful as intermediates for the preparation of novel antibiotics. In this connection, the novel antibiotics are described in, for example, Japanese Patent Application Laid-Open No.308287/1989 (USP 5,128,465).

### Description of the Prior Art:

Some of 2-aminoacetamide derivatives have heretofore been known to be obtained by reacting a secondary amine with 2-chloroacetamide in an aprotic solvent as described in, for example, FR-5423 (FR-1467942), or by reacting a secondary amine with formalin and sodium cyanide and then treating the resulting reaction mixture with hydrochloric acid and subsequently with sulfuric acid as described in Example 1 of Japanese Patent Application Laid-Open No.308287/1989 (USP 5,128,465).

The process described in FR-5423 (FR-1467942) is a preferable process in that the reaction time is as short as 1 to 4 hours. However, this process involves many problems such that since the reaction is conducted under temperature conditions which are heating to reflux in a solvent composed of dimethylformamide (boiling point: 153°C) or dioxane (boiling point: 100-102°C), a starting material cannot be effectively used by evaporation if an amine low in boiling point is used as the starting material, and by-products due to thermal decomposition are formed to a significant extent, and moreover a yield is low (53-83%) if the substituent groups of the amine are other groups than an n-propyl or isoamyl group.

Among commercially-available secondary amines, unsymmetrical amines having different alkyl groups are very few. It has thus been difficult to prepare unsymmetrical 2-aminoacetamide derivatives in accordance with the process described in FR-5423 (FR-1467942).

The process described in Example 1 of Japanese Patent Application Laid-Open No.308287/1989 (USP 5,128,465) has caused uneasiness from the viewpoint of safety in working operations in that sodium cyanide, which is a poison, is used.

As described above, the process described in FR-5423 (FR-1467942) has involved many problems from the viewpoint of economy, product purity, applicability and the like, while the process described in Example 1 of Japanese Patent Application Laid-Open No.308287/1989 (USP 5,128,465) has caused uneasiness from the viewpoint of safety in working operations. There has thus been a demand for development of an industrially excellent process for preparing 2-aminoacetamide derivatives, which can replace these processes. BE-A-885 303 discloses 2-aminoacetamide derivatives of the general formula 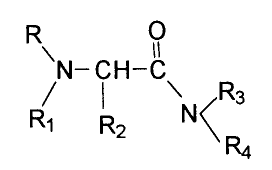 which partially overlaps with the general formula III of the present application. BE-A-885 303 proposes several processes for the production of these compounds including asymmetric embodiments thereof which are based on the use of the corresponding acids or nitriles; of sydnonymins; of isonitriles; or of glyoxal as starting materials.

### SUMMARY OF THE INVENTION

It is thus an object of the present invention to provide an industrially excellent process for preparing 2-aminoacetamide derivatives useful as intermediates for the preparation of novel antibiotics described in Japanese Patent Application Laid-Open No.308287/1989 (USP 5,128,465) and the like.

The present inventors have carried out an extensive investigation with a view toward improving the above-described problems involved in the conventional preparation processes of 2-aminoacetamide derivatives. As a result, it has been found that the desired object can be achieved by any one of the following processes to industrially prepare 2-aminoacetamide derivatives, thus leading to completion of the present invention.

In an aspect of the present invention, there is thus provided a process for preparing a 2-aminoacetamide derivative represented by the following general formula (III): 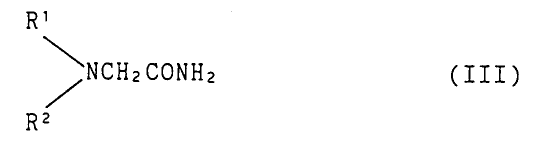 wherein R¹ means a methyl or ethyl group, and R² denotes a linear or branched lower alkyl group having 2-6 carbon atoms, a cycloalkyl group or a phenylalkyl group represented by the following general formula: 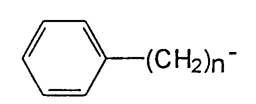 in which n stands for 0 or an integer of 1-2, and may be unsubstituted or substituted, with the proviso that R¹ and R² do not represent an ethyl group at the same time, which comprises
(i) reacting an N-benzylideneamine derivative of the general formula (IV): 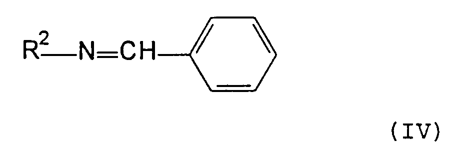 wherein R² has the same meaning as defined above, or a salt thereof with dimethyl sulfate or diethyl sulfate to form a secondary amine of the general formula (I): 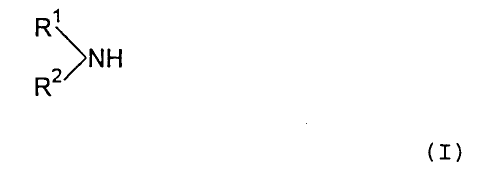 wherein R¹ and R² have the same meaning as defined above, and then
(ii) reacting the secondary amine (I) with a 2-haloacetamide of the general formula (II):

XCH₂CONH₂ (II)

wherein X means a halogen atom, in the presence of at least one solvent selected from water, C₁₋₆ alcohols and aromatic solvents.

In a further aspect of the present invention, there is also provided a process for preparing the 2-aminoacetamide derivative (III), which comprises reacting a primary amine represented by the following general formula (V):

R²-NH₂ (V)

wherein R² has the same meaning as defined above, with benzaldehyde to form the N-benzylideneamine derivative (IV) or a salt thereof, reacting this product with dimethyl sulfate or diethyl sulfate to form the secondary amine (I), and then reacting this secondary amine (I) with the 2-haloacetamide (II).

In yet still a further aspect of the present invention, there is also provided 2-(methyl-n-butylamino)acetamide.

In yet still a further aspect of the present invention, there is also provided 2-(methyl-cyclohexylamino)acetamide.

In still a further aspect of the present invention, there is also provided 2-(ethyl-benzylamino)acetamide.

The above and other objects, features and advantages of the present invention will become apparent from the following description of the preferred examples and the appended claims.

### DETAILED DESCRIPTION OF THE PREFERRED EXAMPLES

A series of the preparation processes according to the present invention can be represented by the following chemical reaction scheme: 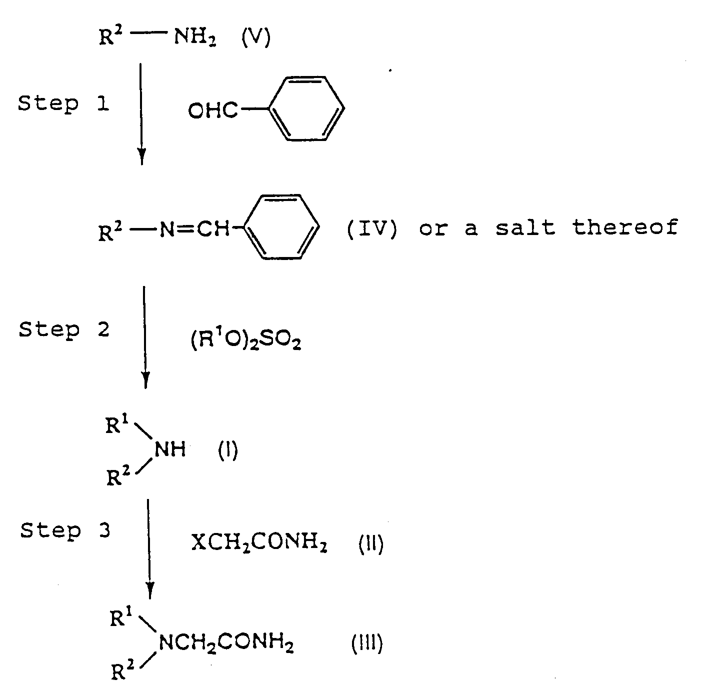 wherein R¹, R² and X have the same meaning as defined above.

Here, the secondary amine (I) useful in the practice of the present invention is represented by the following general formula: 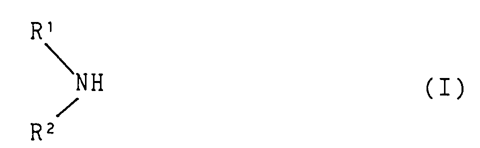 wherein R¹ means a methyl or ethyl group, and R² denotes a linear or branched lower alkyl group having 2-6 carbon atoms, a cycloalkyl group or a phenylalkyl group represented by the following general formula: 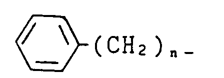 in which n stands for 0 or an integer of 1-2, and may be unsubstituted or substituted.

As specific examples of the secondary amine (I), may be mentioned the following compounds, to which, however, is not limited.
(1) Methyl-ethylamine;
(2) Methyl-n-propylamine;
(3) Methyl-isopropylamine;
(4) Methyl-n-butylamine;
(5) Methyl-isobutylamine;
(6) Methyl-n-amylamine;
(7) Methyl-isoamylamine {CH₃NH[CH₂CH₂CH(CH₃)₂]};
(8) Methyl-sec-amylamine {CH₃NH[CH(CH₃)CH₂CH₂CH₃]};
(9) Methyl-cyclopropylamine;
(10) Methyl-cyclobutylamine;
(11) Methyl-cyclopentylamine;
(12) Methyl-cyclohexylamine;
(13) N-Methylaniline;
(14) N-Methylbenzylamine;
(15) N-Methylphenethylamine;
(17) Ethyl-n-propylamine;
(18) Ethyl-isopropylamine;
(19) Ethyl-n-butylamine;
(20) Ethyl-isobutylamine;
(21) Ethyl-n-amylamine;
(22) Ethyl-isoamylamine;
(23) Ethyl-sec-amylamine;
(24) Ethyl-cyclopropylamine;
(25) Ethyl-cyclobutylamine;
(26) Ethyl-cyclopentylamine;
(27) Ethyl-cyclohexylamine;
(28) N-Ethylaniline;
(29) N-Ethylbenzylamine; and
(30) N-Ethylphenethylamine.

The 2-haloacetamide (II) useful in the practice of the present invention is represented by the following general formula:

XCH₂CONH₂ (II)

wherein X means a halogen atom. As specific examples of the 2-haloacetamide (II), may be mentioned the following compounds.
(1) 2-Chloroacetamide;
(2) 2-Bromoacetamide; and
(3) 2-Iodoacetamide.

The 2-aminoacetamide derivative (III) according to the present invention is represented by the following general formula: 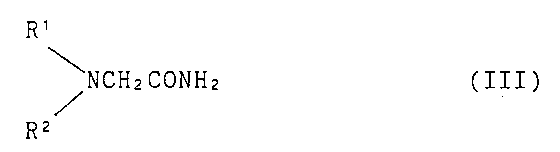 wherein R¹ and R² have the same meaning as defined above. As specific examples of the 2-aminoacetamide (III), may be mentioned the following compounds:
(1) 2-(Methylethylamino)acetamide (N-methyl-N-ethylglycine amide);
(2) 2-(Methyl-n-propylamino)acetamide;
(3) 2-(Methyl-isopropylamino)acetamide;
(4) 2-(Methyl-n-butylamino)acetamide;
(5) 2-(Methyl-isobutylamino)acetamide;
(6) 2-(Methyl-n-amylamino)acetamide;
(7) 2-(Methyl-isoamylamino)acetamide;
(8) 2-(Methyl-sec-amylamino)acetamide;
(9) 2-(Methyl-cyclopropylamino)acetamide;
(10) 2-(Methyl-cyclobutylamino)acetamide;
(11) 2-(Methyl-cyclopentylamino)acetamide;
(12) 2-(Methyl-cyclohexylamino)acetamide;
(13) 2-(Methyl-phenylamino)acetamide;
(14) 2-(Methyl-benzylamino)acetamide;
(15) 2-(Methyl-phenetylamino)acetamide;
(17) 2-(Ethyl-n-propylamino)acetamide;
(18) 2-(Ethyl-isopropylamino)acetamide;
(19) 2-(Ethyl-n-butylamino)acetamide;
(20) 2-(Ethyl-isobutylamino)acetamide;
(21) 2-(Ethyl-n-amylamino)acetamide;
(22) 2-(Ethyl-isoamylamino)acetamide;
(23) 2-(Ethyl-sec-amylamino)acetamide;
(24) 2-(Ethyl-cyclopropylamino)acetamide;
(25) 2-(Ethyl-cyclobutylamino)acetamide;
(26) 2-(Ethyl-cyclopentylamino)acetamide;
(27) 2-(Ethyl-cyclohexylamino)acetamide;
(28) 2-(Ethyl-phenylamino)acetamide;
(29) 2-(Ethyl-benzylamino)acetamide; and
(30) 2-(Ethyl-phenethylamino)acetamide.

The N-benzylideneamine derivative (IV) useful in the practice of the present invention is represented by the following general formula: 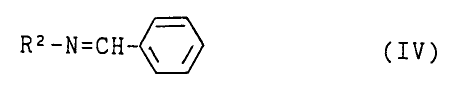 wherein R² has the same meaning as defined above. The N-benzylideneamine derivative (IV) may form a salt in some cases. In the present invention, no limitation is imposed on the form of such an amine derivative, and it may hence be either a free base or a salt. If the salt is formed, no limitation is also imposed on the kind of an acid which forms a counter ion. As specific examples of the salt, however, may be mentioned addition salts with inorganic acids such as hydrochloric acid, hydrobromic acid, hydroiodic acid, sulfuric acid, nitric acid, perchloric acid and phosphoric acid; addition salts with sulfonic acids such as methanesulfonic acid, ethanesulfonic acid, benzenesulfonic acid and toluenesulfonic acid; and addition salts with organic acids such as chloroacetic acid and fluoroacetic acid. As specific examples of the N-benzylideneamine derivative (IV), may be mentioned the following compounds, to which, however, is not limited.
(1) N-Benzylidene-ethylamine;
(2) N-Benzylidene-n-propylamine;
(3) N-Benzylidene-isopropylamine;
(4) N-Benzylidene-n-butylamine;
(5) N-Benzylidene-isobutylamine;
(6) N-Benzylidene-n-amylamine;
(7) N-Benzylidene-isoamylamine;
(8) N-Benzylidene-sec-amylamine;
(9) N-Benzylidene-cyclopropylamine;
(10) N-Benzylidene-cyclobutylamine;
(11) N-Benzylidene-cyclopentylamine;
(12) N-Benzylidene-cyclohexylamine;
(13) N-Benzylideneaniline; and
(14) N-Benzylidene-phenethylamine.

The primary amine (V) useful in the practice of the present invention is represented by the following formula:

R²-NH₂ (V)

wherein R² has the same meaning as defined above. As specific examples of the primary amine (V), may be mentioned the following compound, to which, however, is not limited.
(1) Ethylamine;
(2) n-Propylamine;
(3) Isopropylamine;
(4) n-Butylamine;
(5) Isobutylamine;
(6) n-Amylamine;
(7) Isoamylamine;
(8) sec-Amylamine;
(9) Cyclopropylamine;
(10) Cyclobutylamine;
(11) Cyclopentylamine;
(12) Cyclohexylamine;
(13) Aniline;
(14) Benzylamine; and
(15) Phenethylamine.

No limitation is imposed on the lower alcohol useful in the practice of the present invention so far as it is an alcohol routinely used as a solvent and having carbon atoms not more than 6. As specific examples thereof, however, may be mentioned the following compounds.
(1) Methanol;
(2) Ethanol;
(3) n-Propanol;
(4) Isopropanol;
(5) n-Butanol;
(6) Isobutanol;
(7) tert-Butanol;
(8) Pentanol; and
(9) Hexanol.

Of these, methanol or ethanol is preferred.

No limitation is imposed on the aromatic solvent useful in the practice of the present invention so far as it is an aromatic solvent routinely used as a solvent. As specific examples thereof, however, may be mentioned the following compounds.
(1) Benzene;
(2) Chlorobenzene;
(3) Bromobenzene;
(4) Nitrobenzene;
(5) Anisole;
(6) Toluene;
(7) Xylene;
(8) Ethylbenzene; and
(9) Tetrahydronaphthalene.

Of these, toluene or xylene is preferred.

The preparation processes according to the present invention will hereinafter be described in detail. The present invention can be performed in accordance with any one of the following processes (see the chemical reaction scheme as described above).
(1) A secondary amine (I) is reacted with a 2-haloacetamide (II) in the presence of at least one solvent selected from water, C₁₋₆ alcohols, aromatic solvents and acetic acid esters.
(2) An N-benzylideneamine derivative (IV) or a salt thereof is reacted with dimethyl sulfate or diethyl sulfate to form a secondary amine (I). This secondary amine is then reacted with a 2-haloacetamide (II).
(3) A primary amine (V) is reacted with benzaldehyde to form an N-benzylideneamine derivative (IV) or a salt thereof. This product is then reacted with dimethyl sulfate or diethyl sulfate to form a secondary amine (I). This secondary amine is further reacted with a 2-haloacetamide (II).

### Step 1:

This is a step in which a primary amine (V) is reacted with benzaldehyde to prepare an N-benzylideneamine derivative (IV). This reaction can be performed in accordance with the routine preparation process for a Schiff base in organic synthesis. Since some of the primary amines (V) used as a starting material in the present invention are low in boiling point, it is however preferable that benzaldehyde be added dropwise to the primary amine (V) while cooling the contents so as to keep the internal temperature at 20°C or lower. No limitation is imposed on the amount of benzaldehyde to be used. However, it is generally used in an amount of 0.6-2 equivalents, preferably 0.7-1.5 equivalents, more preferably 0.8-1 equivalent to the primary amine (V).

In this step, a solvent may or may not be used. If the solvent is used, no limitation is imposed on such a solvent so far as it is inert to the primary amine (V) or benzaldehyde. As specific examples thereof, however, may be mentioned the following solvents.

Water, methanol, ethanol, n-propanol, isopropanol, acetone, 2-butanone (methyl ethyl ketone), 3-pentanone (diethyl ketone), 3-hexanone (ethyl propyl ketone), 4-heptanone (dipropyl ketone), 2,4-dimethyl-3-pentanone (diisopropyl ketone), acetonitrile, nitromethane, tetrahydrofuran, 1,2-dimethoxyethane, ethyl ether, isopropyl ether, butyl ether, methyl formate, ethyl formate, methyl acetate, ethyl acetate, propyl acetate, methyl propionate, ethyl propionate, methyl butyrate, ethyl butyrate, 1,4-dioxane, 1,3-dioxolane, formamide, dimethylformamide, dimethyl sulfoxide, hexamethylphosphoramide (HMPA), hexamethylphosphoroustriamide (HMPT), benzene, toluene, xylene, nitrobenzene, chlorobenzene, dichlorobenzene, anisole, pyridine, hexane, octane, decane, decalin, cyclohexane, methylene chloride, chloroform, carbon tetrachloride, 1,2-dichloroethane, 1,1,1-trichloroethane, 1,1,2-trichloroethane, Trichlene, 1,1,1,2-tetrachloroethane, 1,1,2,2-tetrachloroethane, 1-chloropropane, 2-chloropropane, 1,1-dichloropropane, 1,2-dichloropropane, 1,3-dichloropropane and 2,2-dichloropropane.

No limitation is also imposed on the amount of the solvent to be used. However, it is generally used in an amount of about 0.5-100 ml, preferably about 0.7-50 ml, more preferably about 1-20 ml per gram of benzaldehyde. The above-mentioned solvents may be used either singly or in any combination thereof.

Reaction time varies according to the reaction temperature, amounts of benzaldehyde and the solvent to be used, and the like. However, the reaction is generally completed in about 10 minutes to about 6 hours after completion of the addition of benzaldehyde. The N-benzylideneamine derivative (IV) thus formed has a purity sufficient to apply to a next step as it is. However, if a higher purity is required, the reaction mixture may be neutralized to isolate the product, and then further purified by the conventional method such as chromatography on silica gel, HPLC, distillation or recrystallization.

### Step 2:

This is a step in which the N-benzylideneamine derivative (IV) is reacted with dimethyl sulfate or diethyl sulfate to prepare a secondary amine (I).

This reaction can be generally performed in accordance with the process described in Synthesis, 4, 303-305 (1980). In the present invention, it is however preferable that the N-benzylideneamine derivative (IV) is dissolved in a solvent to accelerate the reaction. It is more preferable that dimethyl sulfate or diethyl sulfate be gradually added dropwise at an internal temperature not higher than 60°C. A yield is not lowered even if the freshly distilled dimethyl sulfate or diethyl sulfate is not used as described in the Synthesis. There is also no need to use anhydrous benzene. In addition, the hydrolysis of the resultant quaternary ammonium salt, or dimethyl sulfate or diethyl sulfate may be effected simply by adding water, and there is hence no need to add sulfuric acid as described in the Synthesis.

No limitation is imposed on the solvent used in this reaction so far as it is inert to the N-benzylideneamine derivative (IV), dimethyl sulfate or diethyl sulfate. As specific examples thereof, however, may be mentioned the following solvents:

Acetonitrile, nitromethane, tetrahydrofuran, 1,2-dimethoxyethane, ethyl ether, isopropyl ether, butyl ether, 1,4-dioxane, 1,3-dioxolane, benzene, toluene, xylene, nitrobenzene, chlorobenzene, dichlorobenzene, anisole, pentane, hexane, heptane, octane, nonane, decane, cyclohexane, methylene chloride, chloroform, carbon tetrachloride, 1,2-dichloroethane, 1,1,1-trichloroethane, 1,1,2-trichloroethane, Trichlene, 1,1,1,2-tetrachloroethane, 1,1,2,2-tetrachloroethane, 1-chloropropane, 2-chloropropane, 1,1-dichloropropane, 1,2-dichloropropane, 1,3-dichloropropane and 2,2-dichloropropane.

Of these, hardly soluble solvents in water are preferred, with benzene, toluene or xylene being more preferred.

No limitation is also imposed on the amount of the solvent to be used. However, it is generally used in an amount of about 0.5-100 ml, preferably about 0.7-50 ml, more preferably about 1-20 ml per gram of the N-benzylideneamine derivative (IV). The above-mentioned solvents may be used either singly or in any combination thereof.

No limitation is also imposed on the amount of dimethyl sulfate or diethyl sulfate to be used. However, it is generally used in an amount of 0.7-3 equivalents, preferably 0.8-2 equivalents, more preferably 0.9-1.5 equivalents to the N-benzylideneamine derivative (IV).

Since this reaction is an exothermic reaction, it is preferable that the reaction be conducted under cooling, or while gradually adding dimethyl sulfate or diethyl sulfate dropwise to control the reaction temperature. However, if the internal temperature is too low, the reaction becomes slower. It is hence desirable that the reaction be performed at an internal temperature ranging from 0 to 60°C.

Reaction time varies according to the reaction temperature, amounts of the solvent and dimethyl sulfate or diethyl sulfate to be used, and the like. However, the reaction is generally completed in about 10 minutes to about 6 hours after completion of the addition of dimethyl sulfate or diethyl sulfate.

Incidentally, an isolated secondary amine (I) is obtained by further treating the reaction mixture with a base. The secondary amine (I) thus formed may be purified by the conventional method such as chromatography on silica gel, HPLC, distillation or recrystallization.

### Step 3:

This is a step in which the secondary amine (I) is reacted with a 2-haloacetamide (II) in the presence of at least one solvent selected from water, C₁₋₆ alcohols, aromatic solvents and acetic acid esters to prepare a 2-aminoacetamide derivative (III).

Since this reaction is an exothermic reaction, it is preferable that the secondary amine (I) is dissolved in a solvent, and the 2-haloacetamide (II) is gradually added to the solution under cooling. At this time, it is more desirable from the viewpoint of preventing any rapid reaction and formation of by-products that the internal temperature will be kept at 20°C or lower.

No limitation is imposed on the amount of the solvent to be used. However, it is generally used in such an amount that the concentration (wt.%) of the secondary amine (I) in the solvent is 1-80%, preferably 5-70, more preferably 10-60%.

In this reaction, it is possible to add another solvent. If used, no limitation is imposed on such solvents so far as they are inert to the secondary amine (I) or the 2-haloacetamide (II). As specific examples thereof, however, may be mentioned the following solvents:

Nitromethane, tetrahydrofuran, 1,2-dimethoxyethane, ethyl ether, isopropyl ether, butyl ether, 1,4-dioxane, 1,3-dioxolane, methyl formate, ethyl formate, methyl propionate, ethyl propionate, methyl butyrate, ethyl butyrate, acetone, 2-butanone (methyl ethyl ketone), 3-pentanone (diethyl ketone), 3-hexanone (ethyl propyl ketone), 4-heptanone (dipropyl ketone), 2,4-dimethyl-3-pentanone (diisopropyl ketone), propyl alcohol, butyl alcohol, pentanol, tert-amyl alcohol, formamide, dimethylformamide, dimethyl sulfoxide, hexamethylphosphoramide (HMPA), hexamethylphosphoroustriamide (HMPT), hexane, octane, decane, decalin, cyclohexane, methylene chloride, chloroform, carbon tetrachloride, 1,2-dichloroethane, 1,1,1-trichloroethane, 1,1,2-trichloroethane, Trichlene, 1,1,1,2-tetrachloroethane, 1,1,2,2-tetrachloroethane, 1-chloropropane, 2-chloropropane, 1,1-dichloropropane, 1,2-dichloropropane, 1,3-dichloropropane and 2,2-dichloropropane.

No limitation is also imposed on the amount of the 2-haloacetamide to be used. However, it is generally used in an amount of 0.1-2 equivalents, preferably 0.2-1.5 equivalents, more preferably 0.3-1 equivalent to the secondary amine (I).

In this reaction, an inorganic base such as sodium hydroxide, sodium carbonate, potassium carbonate or sodium hydrogencarbonate, a tertiary organic base such as trimethylamine, triethylamine or N,N-dimethylaniline, or an aromatic base such as pyridine or quinoline may be added to more effectively react the secondary amine (I).

Reaction time varies according to the reaction temperature, amounts of the 2-haloacetamide (II) and solvents to be used, and the like. However, the reaction is generally completed in about 30 minutes to about 12 hours after completion of the addition of the 2-haloacetamide (II).

Incidentally, an isolated 2-aminoacetamide derivative (III) is obtained by further treating the reaction mixture with a base. The 2-aminoacetamide derivative (III) thus formed may be purified by the conventional method such as chromatography on silica gel, HPLC, distillation or recrystallization.

Finally, in order to prepare novel antibiotics by using 2-aminoacetamide derivatives (III) according to the present invention as intermediates for preparation, Examples described in, for example, Japanese Patent Application Laid-Open No. 308287/1989 (USP 5,128,465), and the like may be followed.

The present invention will hereinafter be described specifically by the following Examples.

### Example 1 Synthesis of methyl-ethylamine:

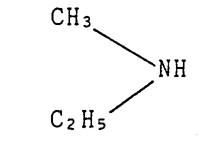

To 100 g (1.553 mol) of a 70% aqueous solution of ethylamine, 150 g (1.415 mol) of benzaldehyde were gradually added dropwise while keeping the internal temperature at 15-20 °C . After the resultant mixture was continuously stirred for 2 hours as it is, a water layer was separated, and 38 g of toluene were added to the residual organic layer, thereby subjecting the organic layer to azeotropic dehydration under reduced pressure (15 mmHg, 60 °C).

To this organic layer thus dehydrated, 214 g (1.698 mol) of dimethyl sulfate were then gradually added dropwise while keeping the internal temperature at 40-50°C. After the resultant mixture was continuously stirred for 2 hours as it is, 141 g of water were gradually added while keeping the internal temperature at 20°C under cooling. The resulting mixture was continuously stirred for 1 hour as it is. An oil layer was separated and removed, and the residual water layer was washed with toluene (47 g x 2). To this water layer, 71 g of water was further added, and the resultant mixture was transferred to a flask equipped with a Claisen column, thereby removing benzaldehyde by steam distillation under ordinary pressure. The distillation residue was gradually added to 328 g of a 50% aqueous solution of sodium hydroxide, which had been cooled, while keeping the internal temperature at 25°C with stirring.

The resultant water layer was then transferred to a Claisen flask to distill it under ordinary pressure, thereby obtaining 75 g of the title compound as a colorless liquid (yield from ethylamine: 81.7%, purity by HPLC: 99 area %).
Boiling point: 36-37°C
Specific gravity: d = 0.69
Refractive index: n_{D}²⁰ = 1.37.

### Example 2 Synthesis of 2-(methyl-ethylamino)acetamide (solvent: water):

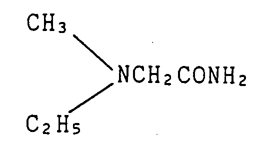

Into 60 g (0.508 mol) of a 50% aqueous solution of methylethylamine, 19.8 g (0.212 mol) of 2-chloroacetamide were gradually added over 3 hours. The resultant mixture was then stirred further for 5 hours at room temperature. The liquid reaction mixture was subjected to extraction with chloroform (30 ml x 4). The resulting extract was washed with brine and then concentrated under reduced pressure. The resultant residue was dried to obtain 22.85 g of the title compound as white crude crystals (yield from 2-chloroacetamide: 92.9%, purity by HPLC: 96.9 area %).
Conditions for HPLC:
Stationary phase: Inertsil ODS-2, 4.6 mm across x 150 mm long;
Mobile phase: 0.5% KH₂PO₄ + 0.2M NaClO₄;
Flow rate: 1.0 ml/min;
Detector: UV 210 nm.

In the following examples, purities were measured in the same manner as described above.

Chloroform (12 g) was added to the crude crystals to dissolve the crystals therein at 50°C. To heptane (66 g) chilled with ice water in advance, the chloroform solution of the crude crystals was gradually added dropwise while chilling with ice water and stirring. The resultant mixture was continuously stirred for 2 hours as it is. Deposited crystals were collected by filtration and dried to obtain 21.3 g of the title compound as white crystals (yield from 2-chloroacetamide: 86.4%).
Melting point: 72.5-73.8°C
¹H-NMR (400 MHz, CDCl₃): δ (ppm)
1.05(3H,t,J=7.1Hz), 2.28(3H,s), 2.49(2H,q,J=7.1Hz), 3.00(2H,s), 6.15(1H,br), 7.10(1H,br).
MS (m/z, M⁺): 116.

### Example 4 Synthesis of 2-(methyl-ethylamino)acetamide (solvent: toluene):

Into 60 g (0.508 mol) of a 50% solution of methylethylamine in toluene, 19.8 g (0.212 mol) of 2-chloroacetamide were gradually added over 3 hours. Thereafter, toluene (10 ml) was added, and the resultant mixture was stirred further for 12 hours at room temperature. The liquid reaction mixture was concentrated to about a half and added with chloroform (120 ml). The resulting mixture was washed with water and then with brine, and then concentrated under reduced pressure. The resultant residue was dried to obtain 18.65 g of the title compound as pale yellow crude crystals (yield from 2-chloroacetamide: 75.8%, purity by HPLC: 98.4 area %).

The crude crystals were purified with chloroform and heptane in the same manner as in Example 2, thereby obtaining 16.8 g of the title compound as white crystals (yield from 2-chloroacetamide: 68.3%).
Melting point: 72.2-73.8°C.

### Example 5 Synthesis of 2-(methyl-ethylamino)acetamide (solvent: methanol):

Into 60 g (0.508 mol) of a 50% solution of methylethylamine in methanol, 19.8 g (0.212 mol) of 2-chloroacetamide were gradually added over 3 hours. The resultant mixture was then stirred further for 12 hours at room temperature. The liquid reaction mixture was concentrated to about a half and added with chloroform (120 ml). The resulting mixture was washed with water and then with brine, and then concentrated under reduced pressure. The resultant residue was dried to obtain 17.98 g of the title compound as pale yellow crude crystals (yield from 2-chloroacetamide: 73.1%, purity by HPLC: 95.6 area %).

The crude crystals were transferred to a Claisen flask equipped with a Vigoureux column (3 cm) to distill them under reduced pressure, thereby obtaining 15.8 g of a fraction having a boiling point of 92-95°C (0.5 mmHg) (yield from 2-chloroacetamide: 64.2%).
Melting point: 72.4-73.8°C.

### Example 6 Synthesis of 2-(methyl-benzylamino)acetamide (solvent: water):

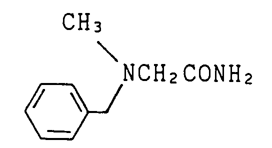

In water (80 ml), were suspended 80 g (0.66 mol) of N-methyl-benzylamine, and 25.7 g (0.28 mol) of 2-chloroacetamide were gradually added to the suspension over 2 hours while keeping the internal temperature at 20-30°C. The resultant mixture was then stirred further for 12 hours at room temperature. The liquid reaction mixture was subjected to extraction with chloroform (100 ml x 2). The resulting extract was washed with brine and then concentrated under reduced pressure, thereby obtaining crystalline residue. The resultant residue was recrystallized from a mixed solution of isopropyl ether and acetone (5:1) to obtain 44.3 g of the title compound as white crystals (yield from 2-chloroacetamide: 90.5 area %, purity by HPLC: 97.5%).
Melting point: 99.3-100.5°C.
¹H-NMR (400 MHz, CDCl₃): δ (ppm)
2.31(3H,s), 3.05(2H,s), 3.60(2H,s), 5.64(1H,br), 6.98(1H,br), 7.24-7.36(5H,m).
MS (m/z, M⁺): 178.

### Example 7 Synthesis of 2-(methyl-benzylamino)acetamide (solvent: water):

In water (80 ml), were suspended 80 g (0.66 mol) of N-methyl-benzylamine, and 25.7 g (0.28 mol) of 2-chloroacetamide were gradually added to the suspension over 2 hours while keeping the internal temperature at 75-85°C. Water (80 ml) was added, and the resultant mixture was stirred further for 12 hours at room temperature. The liquid reaction mixture was subjected to extraction with chloroform (100 ml x 2). The resulting extract was washed with brine and then concentrated under reduced pressure, thereby obtaining crystalline residue. The resultant residue was recrystallized from a mixed solution of isopropyl ether and acetone (5:1) to obtain 37.2 g of the title compound as white crystals (yield from 2-chloroacetamide: 76.0%, purity by HPLC: 97 area %).

### Example 8 Synthesis of 2-(methyl-n-butylamino)acetamide (solvent: water):

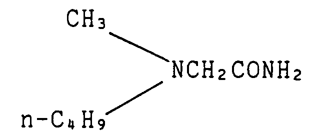

With water (95 ml), were mixed 95.1 g (1.09 mol) of N-methyl-n-butylamine, and 42.5 g (0.45 mol) of 2-chloroacetamide were gradually added to the mixture over 2 hours while keeping the internal temperature at 20-30°C. The resultant mixture was then stirred further for 12 hours at room temperature. The liquid reaction mixture was subjected to extraction with chloroform (100 ml x 4). The resulting extract was washed with brine and then concentrated under reduced pressure, thereby obtaining crystalline residue. The resultant residue was recrystallized from isopropyl ether to obtain 53.8 g of the title compound as white crystals (yield from 2-chloroacetamide: 82.1%, purity by HPLC: 100 area %).
Melting point: 72.3-73.5°C.
¹H-NMR (400 MHz, CDCl₃): δ (ppm)
0.93(3H,t,J=7.2Hz), 1.30-1.39(2H,m), 1.41-1.49(2H,m), 2.30(3H,s) 2.41(2H,t,J=7.3Hz), 2.99(2H,s), 5.92(1H,br), 7.12(1H,br).
MS (m/z, M⁺): 144.

### Example 9 Synthesis of 2-(methyl-cyclohexylamino)acetamide (solvent: water):

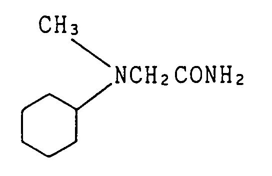

In water (125 ml), were suspended 123.5 g (1.09 mol) of N-methyl-cyclohexylamine, and 42.5 g (0.45 mol) of 2-chloroacetamide were gradually added to the suspension over 2 hours while keeping the internal temperature at 20-30°C. The resultant mixture was then stirred further for 12 hours at room temperature. The liquid reaction mixture was subjected to extraction with chloroform (100 ml x 2). The resulting extract was washed with brine and then concentrated under reduced pressure, thereby obtaining crystalline residue. The resultant residue was recrystallized from isopropyl ether to obtain 62.4 g of the title compound as white crystals (yield from 2-chloroacetamide: 81.5%, purity by HPLC: 98.8 area %).
Melting point: 84.9-85.5°C.
¹H-NMR (400 MHz, CDCl₃): δ (ppm)
1.04-1.30(5H,m), 1.59-1.83(5H,m), 2.30(3H,s), 2.32-2.41(1H,m), 3.04(2H,s), 5.78(1H,br), 7.20(1H,br).
MS (m/z, M⁺): 170.

### Example 10 Synthesis of 2-(ethylbenzylamino)acetamide (solvent: water):

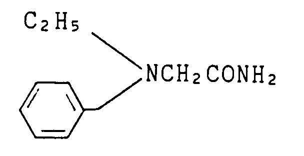

In water (145 ml), were suspended 145.0 g (1.08 mol) of N-ethylbenzylamine, and 42.1 g (0.45 mol) of 2-chloroacetamide were gradually added to the suspension over 2 hours while keeping the internal temperature at 20-30°C. Water (50 ml) was added, and the resultant mixture was stirred further for 1 hour at room temperature. The liquid reaction mixture was subjected to extraction with chloroform (100 ml x 2). The resulting extract was washed with brine and then concentrated under reduced pressure, thereby obtaining crystalline residue. The resultant residue was recrystallized from a mixed solution of isopropyl ether and acetone (2:1) to obtain 63.3 g of the title compound as white crystals (yield from 2-chloroacetamide: 73.5%, purity by HPLC: 99.8 area %).
Melting point: 85.5-86.4°C.
¹H-NMR (400 MHz, CDCl₃): δ (ppm)
1.11(3H,t,J=7.1Hz), 2.60(2H,q,J=7.2Hz), 3.09(2H,s), 3.63(2H,s), 5.39(1H,br), 7.12(1H,br), 7.27-7.40(5H,m).
MS (m/z, M⁺): 192.

## Claims

1. A process for preparing a 2-aminoacetamide derivative of the general formula (III): 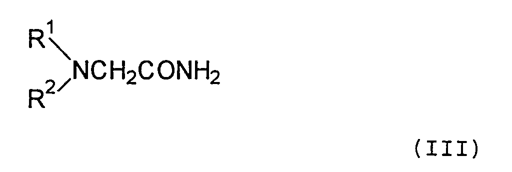 wherein R¹ means a methyl or ethyl group, and R² denotes a linear or branched lower alkyl group having 2-6 carbon atoms, a cycloalkyl group or a phenylalkyl group of the general formula: 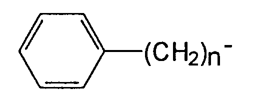 in which n stands for 0 or an integer of 1-2, and may be unsubstituted or substituted, with the proviso that R¹ and R² do not represent an ethyl group at the same time, which comprises
(i) reacting an N-benzylideneamine derivative of the general formula (IV): 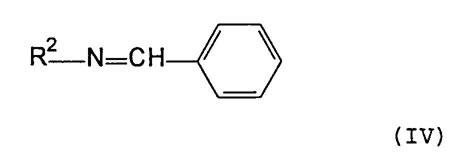 wherein R² has the same meaning as defined above, or a salt thereof with dimethyl sulfate or diethyl sulfate to form a secondary amine of the general formula (I): 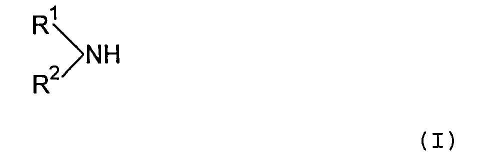 wherein R¹ and R² have the same meaning as defined above, and then
(ii) reacting the secondary amine (I) with a 2-haloacetamide of the general formula (II):
XCH₂CONH₂ (II)
wherein X means a halogen atom, in the presence of at least one solvent selected from water, C₁₋₆ alcohols and aromatic solvents.

2. A process according to claim 1, wherein the N-benzylideneamine derivative (IV) is prepared in a preceding step of reacting an primary amine of the general formula (V):
R²-NH₂ (V)
wherein R² has the same meaning as defined above, with benzaldehyde.

3. The process according to any one of claims 1 to 2 wherein at least one solvent selected from water, C₁₋₆ alcohols and aromatic solvents is used upon the reaction of the secondary amine (I) with the 2-haloacetamide (II).

4. The process according to claim 3, wherein the C₁₋₆ alcohol is methanol or ethanol, or the aromatic solvent is toluene or xylene.

5. The process according to any one of claims 1 to 4, wherein R¹ is a methyl group, and R² is an ethyl group.

6. 2-(Methyl-n-butylamino)acetamide.

7. 2-(Methyl-cyclohexylamino)acetamide.

8. 2-(Ethyl-benzylamino)acetamide.

## Patentansprüche

1. Verfahren zur Herstellung eines 2-Aminoacetamidderivats mit der allgemeinen Formel (III) 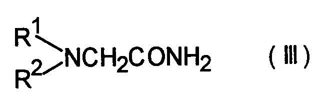 worin R¹ eine Methyl- oder Ethylgruppe bedeutet und R² eine lineare oder verzweigte Niederalkylgruppe mit 2 bis 6 Kohlenstoffatomen, eine Cycloalkylgruppe oder eine Phenylalkylgruppe mit der allgemeinen Formel 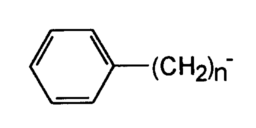 bedeutet, worin n für 0 oder eine ganze Zahl von 1 bis 2 steht, und die substituiert oder unsubstituiert sein kann, mit der Massgabe, dass R¹ und R² nicht gleichzeitig eine Ethylgruppe bedeuten, welches umfasst:
(i) Umsetzung eines N-Benzylidenaminderivats mit der allgemeinen Formel (IV) 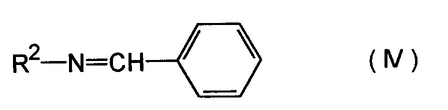 worin R² die gleiche Bedeutung wie oben hat, oder eines Salzes davon mit Dimethylsulfat oder Diethylsulfat, zur Bildung eines sekundären Amins der allgemeinen Formel (I): 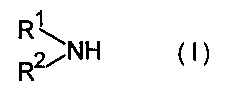 worin R¹ und R² die gleichen Bedeutungen wie oben haben, und dann
(ii) Umsetzung des sekundären Amins (I) mit einem 2-Haloacetamid der allgemeinen Formel (II)
XCH₂CONH₂ (II)
worin X ein Halogenatom bedeutet, in Gegenwart von wenigstens einem Lösungsmittel, ausgewählt aus Wasser, C₁₋₆-Alkoholen und aromatischen Lösungsmitteln.

2. Verfahren gemäss Anspruch 1, worin das N-Benzylidenaminderivat (IV) in einem vorhergehenden Schritt der Umsetzung eines primären Amins der Formel (V)
R²―NH₂ (V)
worin R² die gleiche Bedeutung wie oben hat, mit Benzaldehyd hergestellt wird.

3. Verfahren gemäss einem der Ansprüche 1 bis 2, worin wenigstens ein Lösungsmittel, ausgewählt aus Wasser, C₁₋₆-Alkoholen und aromatischen Lösungsmitteln, bei der Reaktion des sekundären Amins (I) mit dem 2-Haloacetamid (II) verwendet wird.

4. Verfahren gemäss Anspruch 3, worin der C₁₋₆-Alkohol Methanol oder Ethanol ist oder das aromatische Lösungsmittel Toluol oder Xylol ist.

5. Verfahren gemäss einem der Ansprüche 1 bis 4, worin R¹ eine Methylgruppe und R² eine Ethylgruppe ist.

6. 2-(Methyl-n-butylamino)acetamid.

7. 2-(Methyl-cyclohexylamino)acetamid.

8. 2-(Ethyl-benzylamino)acetamid.

## Revendications

1. Procédé de préparation d'un dérivé du 2-aminoacétamide répondant à la formule générale (III) : 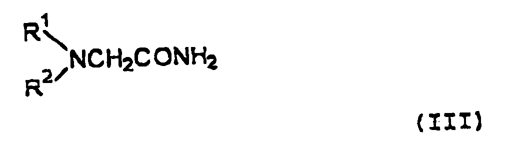 dans laquelle R¹ désigne un groupe méthyle ou éthyle et R² désigne un groupe alkyle inférieur linéaire ou ramifié ayant 2 à 6 atomes de carbone, un groupe cycloalkyle ou un groupe phénylalkyle répondant à la formule générale 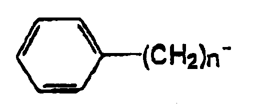 dans laquelle n représente 0 ou un nombre entier égal à 1 ou 2 et peut être non substitué ou substitué, sous réserve que R¹ et R² ne représentent pas simultanément un groupe éthyle, qui comprend
(i) le fait de faire réagir un dérivé de la N-benzylidèneamine répondant à la formule générale (IV) : 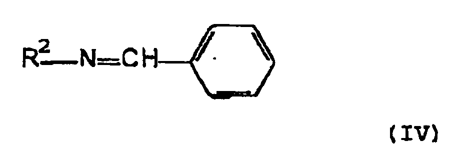 dans laquelle R² a la même signification que ci-dessus, ou un de ses sels, avec le sulfate de diméthyle ou le sulfate de diéthyle pour former une amine secondaire répondant à la formule générale (I) : 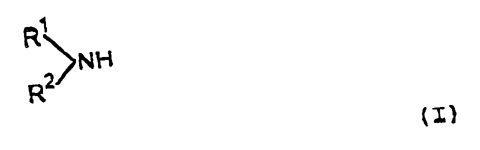 dans laquelle R¹ et R² ont la même signification que ci-dessus, puis
(ii) de faire réagir l'amine secondaire (I) avec un 2-haloacétamide répondant à la formule générale (II) :
XCH₂CONH₂ (II)
dans laquelle X désigne un atome d'halogène, en présence d'au moins un solvant choisi parmi l'eau, des alcools en C₁₋₆ et des solvants aromatiques.

2. Procédé selon la revendication 1, dans lequel le dérivé de la N-benzylidènediamine (IV) est préparé dans une étape précédente consistant à faire réagir une amine primaire répondant à la formule générale (V)
R²-NH₂ (V)
dans laquelle R² a la même signification que ci-dessus, avec le benzaldéhyde.

3. Procédé selon l'une quelconque des revendications 1 à 2, dans lequel on utilise au moins un solvant choisi parmi l'eau, des alcools en C₁₋₆ et des solvants aromatiques lors de la réaction de l'amine secondaire (I) avec le 2-haloacétamide (II).

4. Procédé selon la revendication 3, dans lequel l'alcool en C₁₋₆ est le méthanol ou l'éthanol, ou bien le solvant aromatique est le toluène ou le xylène.

5. Procédé selon l'une quelconque des revendications 1 à 4, dans lequel R¹ est un groupe méthyle et R² est un groupe éthyle.

6. 2-(Méthyl-n-butylamino)acétamide.

7. 2-(Méthyl-cyclohexylamino)acétamide.

8. 2-(Ethyl-benzylamino)acétamide.
